# EUROPEAN PATENT APPLICATION

(11) **EP 4 826 388 A1**
(43) Date of publication of application: **21.10.2026**
(21) Application number: 23961035.5
(22) Date of filing: 11.12.2023
(51) Int. Cl.: G01N 35/10, C12M 1/00, C12Q 1/6869, B05C 9/00

(54) **FLUID PATH AND USE METHOD THEREFOR, FLUID PATH SYSTEM AND USE METHOD THEREFOR, AND SEQUENCER**

(71) Applicant: BGI Shenzhen, Shenzhen, Guangdong 518083 (CN); BGI Shenzhen Co., Ltd, Shenzhen, Guangdong 518083 (CN)
(72) Inventor: QIN, Yanzhe, Shenzhen, Guangdong 518083 (CN); CHEN, Kaijian, Shenzhen, Guangdong 518083 (CN); YU, Shiqiang, Shenzhen, Guangdong 518083 (CN); LUO, Jie, Shenzhen, Guangdong 518083 (CN); LUO, Junjie, Shenzhen, Guangdong 518083 (CN); ZHANG, Wenwei, Shenzhen, Guangdong 518083 (CN); XU, Xun, Shenzhen, Guangdong 518083 (CN); LI, Yuxiang, Shenzhen, Guangdong 518083 (CN); SHEN, Mengzhe, Shenzhen, Guangdong 518083 (CN)
(74) Representative: Bryn Aarflot AS
(86) International application number: PCT/CN2023/137818
(87) International publication number: WO 2025/123168

(57) **Abstract**

Provided are a fluid path and a use method therefor, a fluid path system and a use method therefor, and a sequencer. The fluid path comprises a first surface and a second surface; the first surface at least comprises an exposed chip surface; the first surface and the second surface are arranged at a preset distance; a preset position of the second surface is provided with a reagent fluid; and the second surface can move in a preset direction, and drive the reagent fluid at the preset position to be at least injected into a gap between the chip surface and the second surface. Movement of the second surface drives the reagent fluid to move, thus avoiding using pressure to drive the reagent fluid; the chip surface is in a static state, such that the average flow rate of the fluid path is half of the movement speed of the second surface, and the thickness of the fluid entering the gap will be at least twice the original thickness, thereby reducing the reagent thickness of fluid intake; and the fluid path is an open fluid path, and the first surface and the second surface do not need to be fixed by adhesive bonding, thus overcoming the technical challenge of sealing fluid paths under the condition of small gaps.

## Description

### FIELD

The present application relates to the technical field of gene sequencing, and, in particular, to a fluidic circuit and a method of using the same, a fluidic circuit system and a method of using the same, and a sequencer.

### BACKGROUND

With the development of gene sequencing technology, great progress has been made in gene sequencers in terms of system integration and sequencing time.

A widely used flow cell technology in second-generation gene sequencing methods involves introducing a reagent liquid into a flow cell, which is formed by vertically assembling two planar substrates attached with biological information. Then, base sequence recognition is achieved by performing multiple rounds of imaging on the flow cell. During the sequencing process, the sample loading and fluorescently labeled biochemical reactions are both completed within a gene sequencing chip.

Currently, upper and lower surfaces of a fluidic circuit in the flow cell technology are typically fixed by gluing. The fluidic circuit fixed by gluing has poor reproducibility, a high reagent cost, and a long sequencing time. Furthermore, the flow cell technology drives up costs. The sequencing process requires up to 200 "reaction-imaging" cycles using a variety of reagents. In each cycle, the thickness of the liquid on the chip surface is determined by a gap between the two planar substrates, which is at least 50 micrometers in the related art. However, the reagent layer where the reaction actually occurs is only 1 micrometer near the chip. Consequently, more than 95% of the reagents are wasted.

### SUMMARY

To address the above technical issues, a fluidic circuit and a method of using the same, a fluidic circuit system and a method of using the same, and a sequencer are provided in the present application.

To achieve the above object, the following technical solutions are provided by embodiments of the present application.

A fluidic circuit is provided according to the present application, including a first surface and a second surface.

The first surface includes an exposed chip surface, and is spaced apart from the second surface by a predetermined distance.

The second surface is configured to receive a reagent liquid at a predetermined position, and the second surface is movable in a predetermined direction to drive the reagent liquid at the predetermined position into at least a gap between the chip surface and the second surface.

In some possible embodiments, the predetermined distance is less than or equal to twice a thickness of the reagent liquid at the predetermined position.

In some possible embodiments, the first surface and the second surface are both planar surfaces and arranged parallel to each other, and the predetermined direction is parallel to the first surface.

In some possible embodiments, the first surface and the second surface are both curved surfaces, and the predetermined direction is a clockwise direction or a counterclockwise direction.

In some possible embodiments, a cosine of a contact angle between the first surface and the reagent liquid is greater than 0, and a cosine of a contact angle between the second surface and the reagent liquid is greater than 0.

In some possible embodiments, at least a portion of the second surface is configured as a flexible membrane for contacting the reagent liquid.

Based on the fluidic circuit described above, a method of using the fluidic circuit is further provided according to the present application. The method includes steps of:
applying a reagent liquid to the predetermined position of the second surface of the fluidic circuit; and
controlling the second surface to move in the predetermined direction, so as to drive the reagent liquid at the predetermined position into at least the gap between the chip surface and the second surface.

A fluidic circuit system is further provided according to the present application. The fluidic circuit system includes a stationary module and a reagent transfer module.

The stationary module includes a chip in an exposed state and a first surface. At least a chip surface of the chip is configured as the first surface.

The reagent transfer module is arranged facing the chip. The reagent transfer module has a second surface that is arranged facing the first surface and spaced apart therefrom by a predetermined distance, and the second surface and the first surface define the fluidic circuit described in any one of the above embodiments. The reagent transfer module is configured to receive a reagent liquid at a predetermined position of the second surface, and the reagent transfer module is configured to drive the second surface to move relative to the first surface, so as to drive the reagent liquid at the predetermined position into at least the gap between the chip surface and the second surface.

In some possible embodiments, the reagent transfer module includes a strip and at least two winding-unwinding devices.

The second surface is defined by a surface of the strip arranged facing the first surface.

At least one of the winding-unwinding devices is configured to unwind the strip, and at least one of the winding-unwinding devices is configured to wind the strip.

In some possible embodiments, the fluidic circuit system further includes a positioning device.

The positioning device is configured to locate each of the chip and the reagent transfer module.

In some possible embodiments, the positioning device includes a first suction cup and a second suction cup.

The first suction cup is configured to adsorb the chip.

The second suction cup is arranged opposite to the first suction cup and configured to adsorb the reagent transfer module.

In some possible embodiments, the positioning device further includes a lifting drive mechanism.

A drive end of the lifting drive mechanism is connected to one of the first suction cup and the second suction cup to move the one towards or away from the other of the first suction cup and the second suction cup, so as to adjust a distance between the first surface and the second surface.

In some possible embodiments, the fluidic circuit system further includes a liquid application module.

The liquid application module is configured to apply the reagent liquid onto the predetermined position of the second surface.

In some possible embodiments, the liquid application module includes at least one of a doctor-blade application device, a die-head application device, a spray application device, a spin-coating application device, a screen-printing application device, a needle application device, a dropping application device, and a pipette application device.

Additionally/alternatively, the liquid application module is configured to apply at least one type of reagent liquid onto the predetermined position of the second surface, and the reagent transfer module is configured to, when two or more types of reagent liquid are applied, drive one type of reagent liquid to displace its predecessor, so as to clean and fill the fluidic circuit.

In some possible embodiments, the fluidic circuit system further includes a temperature control device.

The temperature control device is configured to control a temperature of the chip to adjust a temperature of the reagent liquid flowing across the chip surface.

In some possible embodiments, the fluidic circuit system further includes a control unit.

The control unit is communicatively connected to each of electrical components in the fluidic circuit system to control the electrical components.

Based on the fluidic circuit system described above, a method of using the fluidic circuit system is further provided according to the present application. The method includes steps of:
applying a reagent liquid onto the predetermined position of the reagent transfer module; and
controlling the reagent transfer module to move relative to the first surface in the predetermined direction, so as to drive the reagent liquid at the predetermined position into at least a gap between the chip and the reagent transfer module.

In some possible embodiments, the step of applying the reagent liquid to the predetermined position of the reagent transfer module is implemented by at least one of a doctor-blade application device, a die-head application device, a spray application device, a spin-coating application device, a screen-printing application device, a needle application device, a dropping application device, and a pipette application device.

Additionally/alternatively, before the step of applying the reagent liquid to the predetermined position of the reagent transfer module, the method further includes: adsorbing the chip via the first suction cup of the fluidic circuit system, and adsorbing the reagent transfer module via the second suction cup, such that the second surface of the reagent transfer module is spaced apart from the chip surface by a predetermined distance.

Additionally/alternatively, during movement of the reagent liquid driven by the reagent transfer module, the method further includes controlling a temperature of the chip by a temperature control unit of the fluidic circuit system.

A sequencer is further is provided according to the present application, including the fluidic circuit described in any one of the above embodiments or the fluidic circuit system described in any one of the above embodiments.

Through the above technical solutions, the present application has the following beneficial effects.

In the fluidic circuit according to the present application, by applying the reagent liquid onto the predetermined position of the second surface, and then moving the second surface, the reagent liquid at the predetermined position is introduced into the gap between the first surface and the second surface to cover and contact the chip surface for reaction. In the present application, the reagent liquid is driven to move by the movement of the second surface, thereby avoiding using pressure to drive the reagent liquid. Therefore, on one hand, a lower reagent consumption than that in the flow cell technology can be achieved; and on the other hand, there is no issue of exceeding a system pressure limit when a fluid velocity within the flow cell increases. Furthermore, since the reagent liquid is driven to move by the movement of the second surface while the chip surface remains stationary, the average flow velocity in the fluidic circuit is half of the moving speed of the second surface. Since an inflowing velocity of the liquid is equal to the moving speed of the second surface, the thickness of the liquid driven into the gap between the first surface and the second surface can be accommodated up to at least twice an initial thickness thereof, thereby reducing the required initial thickness of the applied reagent liquid. Additionally, the fluidic circuit is an open fluidic circuit, and there is no need for gluing between the first surface and the second surface for fixation. Therefore, there is no need to apply adhesive on one of the first surface and the second surface, and there is no adhesive layer between the first surface and the second surface. That is, the present application eliminates the adhesive layer that would otherwise occupy a certain height of the gap, thus overcoming the process obstacle of sealing a fluidic circuit under a small gap. Moreover, in the prior art, a solid wall is employed in the fluidic circuit, which causes retention of components of the reagent liquid on the solid wall and results in a prolonged retention time for each component of the reagent liquid, thereby requiring a larger amount of a subsequent reagent liquid for flushing before the sequencing reaction can start. In contrast, in the present application, the fluidic circuit is formed by the second surface and the first surface, and the reagent liquid moves along with the movement of the second surface, so that, after completing the reaction with the chip, the reagent liquid may be moved out of the first surface along with the movement of the second surface, thereby avoiding the issue where the reagent liquid retains on the solid wall and requires a large amount of a subsequent reagent liquid for cleaning.

### BRIEF DESCRIPTION OF THE DRAWINGS

To more clearly illustrate solutions in embodiments of the present application or in the related art, drawings required in description of the embodiments or the related art will be briefly introduced below. Apparently, the drawings in the following description only relate to some embodiments of the present application, and other drawings can be obtained for those skilled in the art based on these drawings without creative efforts.
FIG. 1 is a schematic view of a fluidic circuit system according to an embodiment of the present application;
FIG. 2 is a schematic view of a planar-surface fluidic circuit system according to an embodiment of the present application;
FIG. 3 is a schematic view of a dual-curved-surface fluidic circuit system according to an embodiment of the present application;
FIG. 4 is a schematic view of a pipette-type fluidic circuit system according to an embodiment of the present application;
FIG. 5 is a schematic view of a doctor blade-type fluidic circuit system according to an embodiment of the present application;
FIG. 6 is a schematic view of a needle-type fluidic circuit system according to an embodiment of the present application; and
FIG. 7 is a schematic view of a spray-type fluidic circuit system according to an embodiment of the present application.

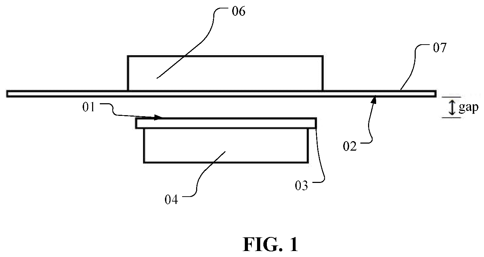

Reference numerals in FIG. 1 to FIG. 7 are listed as follows:

| | | | |
|---|---|---|---|
| 01. | first surface; | 02. | second surface; |
| 03. | chip; | 04. | chip positioning reference; |
| 05. | reagent liquid; | 05a. | first reagent liquid; |
| 05b. | second reagent liquid; | 06. | moving surface positioning reference; |
| 08. | liquid application module; | 07. | reagent transfer module. |

### DETAILED DESCRIPTION OF THE EMBODIMENTS

To facilitate a better understanding of the solutions disclosed in the embodiments of the present application, an application scenario of the solutions is introduced before describing the methods according to the embodiments of the present application.

A currently widely used flow cell technology in second-generation gene sequencing methods involves introducing a reagent liquid into a flow cell, which is formed by vertically assembling two planar substrates attached with biological information. Then, base sequence recognition is achieved by performing multiple rounds of imaging on the flow cell formed by vertically assembling the two planar substrates attached with biological information. Current second-generation sequencing technologies mainly include fluorescent labeling methods based on optical detection and chemical sequencing methods that identify bases through changes in hydrogen ion concentration. The chemical sequencing methods based on changes in hydrogen ion concentration have advantages in detection instrument size and speed, but suffers from homopolymer errors and low throughput. The second-generation sequencing technology based on optical detection for identification offers the highest base identification accuracy and currently the largest throughput. In the sequencing field, a flow cell is generally also referred to as a flow channel, reaction cell, chip, sequencing chip, gene sequencing chip, or colloidal gold, and common English names thereof include FlowCell, Flowcell, Chip, Chip Kit, and Cartridge, etc. During the sequencing process, sample loading and fluorescently labeled biochemical reactions are both completed within the gene sequencing chip. Currently, a system surface and a lower surface of a fluidic circuit system in the flow cell technology are typically fixed by gluing. The fluidic circuit system fixed by gluing has poor reproducibility, high reagent cost, and a long sequencing time.

To address the above technical issues, a fluidic circuit is provided in the present application. Specifically, the fluidic circuit includes a first surface 01 and a second surface 02.

The first surface 01 includes an exposed chip surface of a chip 03. Specifically, the exposed chip surface of the chip 03 refers to a surface of the chip 03 to be sequenced, which is used for reaction and has been loaded with a DNB (DNA nanoball) library.

It should be noted that the exposed chip surface of the chip 03 may also be a surface of the chip 03 to be sequenced loaded with other substances.

It should be further noted that the first surface 01 may be entirely the exposed chip surface of the chip 03, or may include, in addition to the exposed chip surface of the chip 03, a peripheral protective surface or the like arranged at a position not participating in the reaction to protect the chip surface of the chip 03.

A reagent liquid 05 is provided at a predetermined position of the second surface 02. The reagent liquid 05 can move along with the movement the second surface 02.

The second surface 02 can move in a predetermined direction to drive the reagent liquid 05 at the predetermined position into at least the gap between the chip surface of the chip 03 and the second surface 02. In the present application, by driving the reagent liquid 05 into at least the gap between the chip surface of the chip 03 and the second surface 02, the reagent liquid 05 is made to at least flow over the chip surface of the chip 03 to complete the sequencing.

It should be noted that the reagent liquid 05 may be applied onto the predetermined position of the second surface 02 by a liquid application device. Specific types of reagent liquid 05 to be applied are sequentially loaded onto the predetermined position according to a reaction sequence. Through the movement of the second surface 02, each type of reagent liquid 05 may be cleaned and replaced by a subsequent type of the reagent liquid 05 driven into the fluidic circuit.

Referring to FIG. 1, the first surface 01 may be located above the second surface 02, or below the second surface 02. FIG. 1 provided in the embodiment of the present application shows only an exemplary position for illustration, and the embodiments of the present application are not limited thereto. The reagent liquid 05 in the gap between the first surface 01 and the second surface 02 is used for detecting a gene sequence. Specifically, the reagent liquid 05 comes into contact with the exposed chip surface of the chip 03 to react, thereby achieving identification of a base sequence in a gene.

During a gene sequencing process, the reagent liquid 05 flows across different positions on the chip surface of the chip 03 along with the movement of the second surface 02 to perform reactions. As an example, the sequencing process may require replacing various reagent liquids 05 in the gap to achieve up to 200 "reaction-imaging" cycles. In the embodiment of the present application, one type of reagent liquid 05 is applied onto the predetermined position of the second surface 02. Then, the second surface 02 moves in the predetermined direction V, driving the reagent liquid 05 at the predetermined position into the gap between the first surface 01 and the second surface 02. Moreover, a portion of the second surface 02 originally located above the first surface 01 moves out from above the first surface 01, so that the reagent liquid 05 in the gap adhering to the second surface 02 is drawn out from the gap along with the movement of the second surface 02. With the continuous injection of the new reagent liquid 05 at the predetermined position and the continuous extraction of the original reagent liquid 05 from the gap, the reagent liquid 05 in the gap is gradually replaced by the new reagent liquid 05 to complete the cleaning and filling of the fluidic circuit, thereby achieving displacement of the reagent liquid 05 in contact with the sequencing chip 03. For convenience of description, the original reagent liquid 05 is referred to as a first reagent liquid 05a, and the new reagent liquid 05 is referred to as a second reagent liquid 05b.

In the embodiment of the present application, when the first reagent liquid 05a in the gap between the first surface 01 and the second surface 02 is completely displaced by the second reagent liquid 05b, the first reagent liquid 05a has already reacted, and the reaction result has been recorded by imaging. At this point, another type of reagent liquid 05 may be continuously provided while the second surface 02 continues to move, such that the second reagent liquid 05b will replace the first reagent liquid 05a in the gap between the first surface 01 and the second surface 02, thereby achieving multiple "reaction-imaging" cycles and obtaining multiple gene sequencing results.

To ensure that the gap between the first surface 01 and the second surface 02 is completely filled with the reagent liquid 05, in the embodiment of the present application, a height of the gap between the first surface 01 and the second surface 02 is less than or equal to twice the thickness of the reagent liquid 05 at the predetermined position. It should be noted that during the movement of the second surface 02, an inflow rate of the reagent liquid to be driven into the gap is equal to the moving speed of the second surface 02 multiplied by the liquid thickness and the liquid width at the predetermined position on the first surface 01. Since the first surface 01 is stationary, the average flow velocity of the reagent liquid 05 in the gap is half of the moving speed of the second surface 02. Therefore, the flow rate of the reagent liquid 05 in the gap is equal to the moving speed of the second surface 02 multiplied by half of the gap height and the width of the reagent liquid 05. Accordingly, to ensure that the flow rate of the reagent liquid 05 in the gap is less than or equal to the inflow rate of the liquid, i.e., to ensure the gap between the first surface 01 and the second surface 02 is completely filled with the reagent liquid 05, the height of the gap between the first surface 01 and the second surface 02 is less than or equal to twice the thickness of the reagent liquid 05 at the predetermined position.

In the embodiments of the present application, wetting properties of the first surface 01 and the second surface 02 meet the requirements of the reagent liquid 05 to be driven into the gap. A cosine of a contact angle between the first surface 01 and the reagent liquid 05 is greater than 0, and a cosine of a contact angle between the second surface 02 and the reagent liquid 05 is greater than 0.

At least a portion of the second surface 02 is configured as a windable flexible membrane for contacting the reagent liquid 05. In practical applications, the second surface 02 needs to move in the predetermined direction multiple times. In the embodiments of the present application, a portion of the second surface 02 that has moved out of the gap can be wound up and stored.

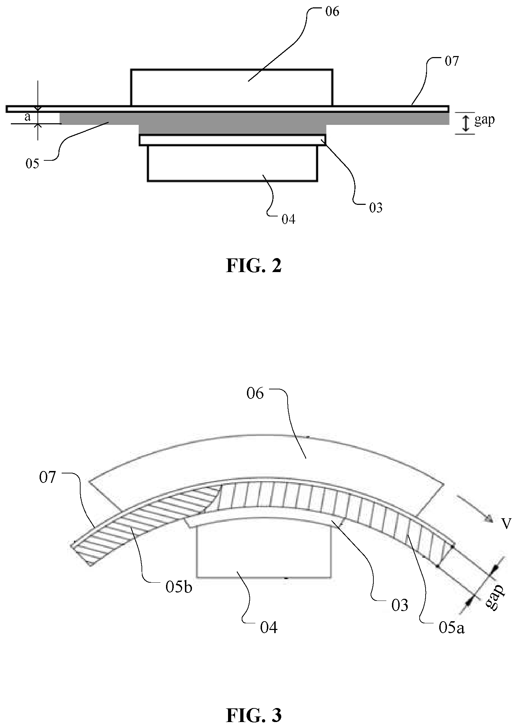

Referring to FIG. 2, as a possible implementation, the first surface 01 and the second surface 02 may both be planar surfaces, and the first surface 01 is arranged parallel to the second surface 02. A distance between the first surface 01 and the second surface 02, i.e., a gap height between the first surface 01 and the second surface 02, is less than or equal to twice the thickness a of the reagent liquid 05 at the predetermined position. The predetermined direction V is parallel to the first surface 01. The first surface 01 is connected to a chip positioning reference 04, and the second surface 02 is connected to a moving surface positioning reference 06. In practical applications, the first surface 01 and the chip positioning reference 04 may be connected via a first suction cup, and the second surface 02 and the moving surface positioning reference 06 may be connected via a second suction cup. It should be noted that the chip positioning reference 04 may be an adsorption surface of the first suction cup for adsorbing the first surface 01, or may be a separately provided positioning block having a surface serving as the chip positioning reference 04, with one end of the first suction cup fixed on the positioning block and the other end thereof configured to adsorb the first surface 01. Similarly, the moving surface positioning reference 06 may be an adsorption surface of the second suction cup for adsorbing the second surface 02, or may be a separately provided positioning block having a surface serving as the moving surface positioning reference 06, with one end of the second suction cup fixed on the positioning block and the other end thereof configured to adsorb the second surface 02.

Referring to FIG. 3, as another possible implementation, the first surface 01 and the second surface 02 are both curved surfaces, and the predetermined direction V is a clockwise direction or a counterclockwise direction. The gap height between the first surface 01 and the second surface 02 is less than or equal to twice the thickness of the reagent liquid 05 at the predetermined position. The reagent liquid 05 at the predetermined position on the first surface 01 is identical to the reagent liquid 05 in the gap between the first surface 01 and the second surface 02. When the second surface 02 moves in the predetermined direction, the reagent liquid 05 at the predetermined position displaces the reagent liquid 05 in the gap between the first surface 01 and the second surface 02. The first surface 01 is connected to the chip positioning reference 04, and the second surface 02 is connected to the moving surface positioning reference 06. In practical applications, the first surface 01 and the chip positioning reference 04 may be connected via a first suction cup, and the second surface 02 and the moving surface positioning reference 06 may be connected via a second suction cup.

A method of using the fluidic circuit described in any one of the above embodiments is further provided according to the present application, which includes the following steps.

In step S1, the reagent liquid 05 is applied to the predetermined position of the second surface 02 of the fluidic circuit.

In step S2, the second surface 02 is controlled to move in the predetermined direction, so as to drive the reagent liquid 05 at the predetermined position into at least the gap between the chip surface of the chip 03 and the second surface 02.

A fluidic circuit system is further provided according to the present application. Specifically, the fluidic circuit system includes a stationary module and a reagent transfer module 07.

The stationary module includes a chip 03 in an exposed state. That is, the stationary module may only include the chip 03 in the exposed state, or may include, in addition to the chip 03 in the exposed state, a protective member surrounding a periphery of the chip 03.

The stationary module has a first surface 01, and at least the chip surface of the chip 03 is configured as the first surface 01.

The reagent transfer module 07 is arranged facing the chip 03. The reagent transfer module 07 has a second surface 02 that is arranged facing the first surface 01 and spaced apart therefrom by a predetermined distance. The second surface 02 and the first surface 01 define the fluidic circuit as described in any one of the above embodiments.

The reagent transfer module 07 is configured to receive a reagent liquid 05 at a predetermined position of the second surface 02. The reagent transfer module 07 is configured to drive the second surface 02 to move relative to the first surface 01, so as to drive the reagent liquid 05 at the predetermined position into at least the gap between the chip surface of the chip 03 and the second surface 02.

In the present application, the reagent liquid 05 is driven to move by the movement of the reagent transfer module 07, thereby avoiding using pressure to drive the reagent liquid. Therefore, on one hand, a lower reagent consumption than that in the flow cell technology can be achieved; and on the other hand, there is no issue of exceeding a system pressure limit when a fluid velocity within the flow cell increases. Furthermore, since the reagent liquid 05 is driven to move by the movement of the reagent transfer module 07 while the chip surface of the chip 03 remains stationary, the average flow velocity in the fluidic circuit is half of the moving speed of the reagent transfer module 07. Since an inflowing velocity of the liquid is equal to the moving speed of the reagent transfer module 07, the thickness of the liquid driven into the gap between the first surface 01 and the second surface 02 can be accommodated up to at least twice an initial thickness thereof, thereby reducing the required initial thickness of the applied reagent liquid. Additionally, since there is no need for gluing between the reagent transfer module 07 and the stationary module for fixation, there is no need to apply adhesive on one of the first surface 01 and the second surface 02, and there is no adhesive layer between the first surface 01 and the second surface 02. That is, the present application eliminates the adhesive layer that would otherwise occupy a certain height of the gap, thus overcoming the process obstacle of sealing a fluidic circuit under a small gap. Moreover, in the related art, a solid wall is employed in the fluidic circuit, which causes retention of components of the reagent liquid 05 on the solid wall and results in a prolonged retention time for each component of the reagent liquid 05, thereby requiring a larger amount of a subsequent reagent liquid 05 for flushing before the sequencing reaction can start. In contrast, in the present application, the fluidic circuit is formed by the second surface 02 and the first surface 01, and the reagent liquid 05 moves along with the movement of the second surface 02, so that, after completion of the reaction with the chip 03, the reagent liquid 05 may be moved out of the first surface 01 along with the movement of the second surface 02, thereby avoiding the issue where the reagent liquid 05 retains on the solid wall and requires a large amount of a subsequent reagent liquid 05 for cleaning.

In some embodiments, the reagent transfer module 07 includes a strip and a winding-unwinding device. A surface of the strip arranged facing the first surface 01 is configured as the second surface 02.

There are at least two winding-unwinding devices. At least one of the winding-unwinding devices is configured to unwind the strip, and at least one of the winding-unwinding devices is configured to wind the strip. The provision of the winding-unwinding devices facilitates the winding and unwinding of the strip, thereby achieving high convenience and efficiency.

In some embodiments, the fluidic circuit system further includes a positioning device. The positioning device is configured to locate each of the chip 03 and the reagent transfer module 07.

Specifically, the positioning device includes a first suction cup and a second suction cup. The first suction cup is configured to adsorb the chip 03. The second suction cup is arranged opposite to the first suction cup and is configured to adsorb the reagent transfer module 07.

It should be noted that the specific structure of the positioning device is not limited to the above structure and may also be other structures.

Further, the positioning device further includes a lifting drive mechanism. A drive end of the lifting drive mechanism is connected to one of the first suction cup and the second suction cup to move the one towards or away from the other of the first suction cup and the second suction cup, so as to adjust the distance between the first surface 01 and the second surface 02.

Specifically, the lifting drive mechanism may be a lead screw nut or an electric pusher, etc.

In some embodiments, the fluidic circuit system further includes a liquid application module 08. The liquid application module 08 is configured to apply the reagent liquid 05 onto the predetermined position of the second surface 02.

Further, the liquid application module 08 includes at least one of a doctor-blade application device, a die-head application device, a spray application device, a spin-coating application device, a screen-printing application device, a needle application device, a dropping application device, and a pipette application device.

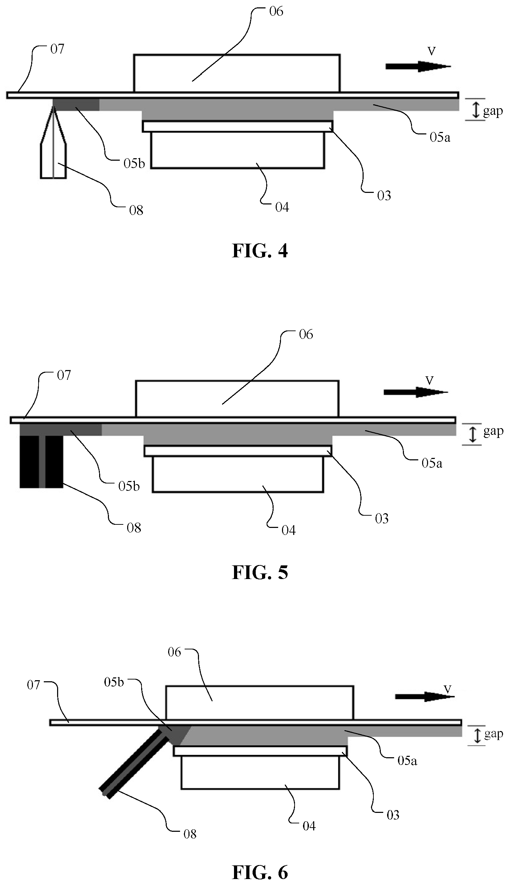

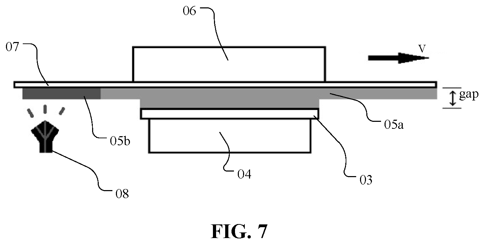

As shown in FIG. 4, the liquid application module 08 in the fluidic circuit system provided in an embodiment of the present application is a pipette application module 08. As shown in FIG. 5, the liquid application module 08 in the fluidic circuit system provided in an embodiment of the present application is a doctor-blade application module 08. As shown in FIG. 6, the liquid application module 08 in the fluidic circuit system provided in an embodiment of the present application is a needle application module 08. As shown in FIG. 7, the liquid application module 08 in the fluidic circuit system provided in an embodiment of the present application is a spray application module 08. The above-mentioned liquid application modules 08 in the embodiments of the present application are only examples and should not be construed as limiting the liquid application module 08 in the embodiments of the present application. In fact, any industrial device capable of applying the reagent liquid 05 to a predetermined position may be applied to the technical solution of the present application. The application methods in the present application are not limited to being implemented by means of a doctor blade, a die head, spraying, spin coating, screen printing, dropping, etc.

In some embodiments, the liquid application module 08 is configured to apply at least one type of reagent liquid 05 onto the predetermined position of the second surface 02, and the reagent transfer module 07 is configured to, when two or more types of reagent liquid 05 are applied, drive one type of reagent liquid 05 to displace its predecessor, so as to clean and fill the fluidic circuit.

In the fluidic circuit system provided in the embodiments of the present application, the fluidic circuit system further includes a temperature control device. The temperature control device is configured to control the temperature of the chip 03 to adjust the temperature of the reagent liquid flowing across the chip surface of the chip 03.

It should be noted that during the actual reaction process, it may be necessary to control the temperature of the chip 03 to be sequenced. After the gap between the first surface 01 and the second surface 02 is filled with the reagent liquid 05, the temperature control device in the embodiment of the present application may adjust the temperature of the chip 03 to be sequenced, thereby achieving the sequencing reaction and obtaining the reaction result.

Further, the fluidic circuit system further includes a control unit. The control unit is communicatively connected to each of electrical components in the fluidic circuit system to control the electrical components. Specifically, the electrical components include, but are not limited to, the temperature control device, the liquid application module 08, the reagent transfer module 07, and the positioning device.

A method of using the fluidic circuit system described in any one of the above embodiments is further provided according to the present application, which includes the following steps.

In step S10, a reagent liquid 05 is applied onto a predetermined position of the reagent transfer module 07.

Specifically, the reagent liquid 05 is applied onto the predetermined position by the liquid application module 08. More specifically, the liquid application module 08 includes at least one of a doctor-blade application device, a die-head application device, a spray application device, a spin-coating application device, a screen-printing application device, a needle application device, a dropping application device, and a pipette application device.

In step S20, the reagent transfer module 07 is controlled to move relative to the first surface 01 in the predetermined direction, so as to drive the reagent liquid 05 at the predetermined position into at least a gap between the chip 03 and the reagent transfer module 07.

Specifically, during movement of the reagent liquid 05 driven by the reagent transfer module 07, the method further includes controlling the temperature of the chip 03 via the temperature control unit of the fluidic circuit system.

It should be noted that controlling the reagent transfer module 07 to drive the reagent liquid 05 at the predetermined position into at least the gap between the chip 03 and the reagent transfer module 07 includes: when only one type of reagent liquid 05 is present, filling the gap with this type of reagent liquid 05; when at least two types of reagent liquid 05 are present (the at least two types of reagent liquid 05 include at least two types of reagent liquid 05 in a single sequencing cycle, and at least two types of reagent liquid 05 that alternate between two consecutive test cycles), the reagent transfer module 07 is capable of driving one type of reagent liquid 05 to displace its predecessor, so as to clean and fill the fluidic circuit.

Further, the present application discloses that, before step S10, the method further includes step S30: adsorbing the chip 03 via the first suction cup of the fluidic circuit system, and adsorbing the reagent transfer module 07 via the second suction cup, such that the second surface 02 of the reagent transfer module 07 is spaced apart from the chip surface of the chip 03 by a predetermined distance.

Specifically, the predetermined distance is less than or equal to twice the thickness of the reagent liquid 05 at the predetermined position.

A sequencer is further provided according to the present application, including the fluidic circuit as described in any one of the above embodiments or the fluidic circuit system as described in any one of the above embodiments.

From the description of the above implementations, those skilled in the art can clearly understand that all or part of the steps in the methods of the above embodiments may be implemented by software plus the necessary general hardware platform. Based on such understanding, the technical solution of the present application essentially, or the part contributing to the prior art, may be embodied in the form of a software product. The computer software product may be stored in a storage medium, such as ROM/RAM, magnetic disk, optical disk, etc., and includes several instructions for causing a computer device (which can be a personal computer, a server, or a network communication device such as a media gateway, etc.) to execute the methods described in various embodiments or certain parts of the embodiments of the present application.

It should be noted that the various embodiments in this specification are described in a progressive manner, with each embodiment focusing on explaining the differences from other embodiments, and the same or similar parts among the various embodiments may refer to each other. For the methods disclosed in the embodiments, since they correspond to the systems disclosed in the embodiments, the description thereof is relatively brief, and for the relevant details, reference may be made to the description of the systems.

It should also be noted that in this document, the terms "comprising", "including", or any other variation thereof are intended to cover non-exclusive inclusion, so that a process, method, article, or device including a series of elements includes not only those elements but also other elements not expressly listed, or elements inherent to such process, method, article, or device. Without further limitations, an element defined by the phrase "comprising a ..." does not preclude the presence of additional identical elements in the process, method, article, or device that includes the element.

The foregoing description of the disclosed embodiments enables those skilled in the art to practice or use the present application. Various modifications to these embodiments will be apparent to those skilled in the art, and the general principles defined herein may be implemented in other embodiments without departing from the spirit or scope of the present application. Accordingly, the present application is not be limited to the embodiments described herein, but encompasses the widest scope consistent with the principles and novel features disclosed herein.

## Claims

1. A fluidic circuit, **characterized by** comprising a first surface and a second surface;
wherein the first surface comprises an exposed chip surface, and is spaced apart from the second surface by a predetermined distance; and
wherein the second surface is configured to receive a reagent liquid at a predetermined position, and the second surface is movable in a predetermined direction to drive the reagent liquid at the predetermined position into at least a gap between the chip surface and the second surface.

2. The fluidic circuit according to claim 1, wherein the predetermined distance is less than or equal to twice a thickness of the reagent liquid at the predetermined position.

3. The fluidic circuit according to claim 1, wherein the first surface and the second surface are both planar surfaces and arranged parallel to each other, and the predetermined direction is parallel to the first surface.

4. The fluidic circuit according to claim 1, wherein the first surface and the second surface are both curved surfaces, and the predetermined direction is a clockwise direction or a counterclockwise direction.

5. The fluidic circuit according to claim 1, wherein a cosine of a contact angle between the first surface and the reagent liquid is greater than 0, and a cosine of a contact angle between the second surface and the reagent liquid is greater than 0.

6. The fluidic circuit according to any one of claims 1 to 5, wherein at least a portion of the second surface is configured as a flexible membrane for contacting the reagent liquid.

7. A method of using the fluidic circuit according to any one of claims 1 to 6, **characterized by** comprising steps of:
applying a reagent liquid to the predetermined position of the second surface of the fluidic circuit; and
controlling the second surface to move in the predetermined direction, so as to drive the reagent liquid at the predetermined position into at least the gap between the chip surface and the second surface.

8. A fluidic circuit system, **characterized by** comprising:
a stationary module, comprising a chip in an exposed state and a first surface, wherein at least a chip surface of the chip is configured as the first surface; and
a reagent transfer module arranged facing the chip, wherein the reagent transfer module has a second surface that is arranged facing the first surface and spaced apart therefrom by a predetermined distance, and the second surface and the first surface define the fluidic circuit according to any one of claims 1 to 6,
wherein the reagent transfer module is configured to receive a reagent liquid at a predetermined position of the second surface, and the reagent transfer module is configured to drive the second surface to move relative to the first surface, so as to drive the reagent liquid at the predetermined position into at least the gap between the chip surface and the second surface.

9. The fluidic circuit system according to claim 8, wherein the reagent transfer module comprises:
a strip, wherein the second surface is defined by a surface of the strip arranged facing the first surface; and
at least two winding-unwinding devices, wherein at least one of the winding-unwinding devices is configured to unwind the strip, and at least one of the winding-unwinding devices is configured to wind the strip.

10. The fluidic circuit system according to claim 8, further comprising a positioning device,
wherein the positioning device is configured to locate each of the chip and the reagent transfer module.

11. The fluidic circuit system according to claim 10, wherein the positioning device comprises:
a first suction cup configured to adsorb the chip; and
a second suction cup arranged opposite to the first suction cup and configured to adsorb the reagent transfer module.

12. The fluidic circuit system according to claim 11, wherein the positioning device further comprises a lifting drive mechanism,
wherein a drive end of the lifting drive mechanism is connected to one of the first suction cup and the second suction cup to move the one towards or away from the other of the first suction cup and the second suction cup, so as to adjust a distance between the first surface and the second surface.

13. The fluidic circuit system according to claim 8, further comprising a liquid application module,
wherein the liquid application module is configured to apply the reagent liquid onto the predetermined position of the second surface.

14. The fluidic circuit system according to claim 13, wherein the liquid application module comprises at least one of a doctor-blade application device, a die-head application device, a spray application device, a spin-coating application device, a screen-printing application device, a needle application device, a dropping application device, and a pipette application device; and/or
wherein the liquid application module is configured to apply at least one type of reagent liquid onto the predetermined position of the second surface, and the reagent transfer module is configured to, when two or more types of reagent liquid are applied, drive one type of reagent liquid to displace its predecessor, so as to clean and fill the fluidic circuit.

15. The fluidic circuit system according to claim 8, further comprising a temperature control device,
wherein the temperature control device is configured to control a temperature of the chip to adjust a temperature of the reagent liquid flowing across the chip surface.

16. The fluidic circuit system according to any one of claims 8 to 15, further comprising a control unit,
wherein the control unit is communicatively connected to each of electrical components in the fluidic circuit system to control the electrical components.

17. A method of using the fluidic circuit system according to any one of claims 8 to 16, **characterized by** comprising steps of:
applying a reagent liquid onto the predetermined position of the reagent transfer module; and
controlling the reagent transfer module to move relative to the first surface in the predetermined direction, so as to drive the reagent liquid at the predetermined position into at least a gap between the chip and the reagent transfer module.

18. The method according to claim 17, wherein the step of applying the reagent liquid to the predetermined position of the reagent transfer module is implemented by at least one of a doctor-blade application device, a die-head application device, a spray application device, a spin-coating application device, a screen-printing application device, a needle application device, a dropping application device, and a pipette application device; and/or
wherein before the step of applying the reagent liquid to the predetermined position of the reagent transfer module, the method further comprises: adsorbing the chip via the first suction cup of the fluidic circuit system, and adsorbing the reagent transfer module via the second suction cup, such that the second surface of the reagent transfer module is spaced apart from the chip surface by a predetermined distance; and/or
during movement of the reagent liquid driven by the reagent transfer module, the method further comprises controlling a temperature of the chip by a temperature control unit of the fluidic circuit system.

19. A sequencer, **characterized by** comprising the fluidic circuit according to any one of claims 1 to 6 or the fluidic circuit system according to any one of claims 8 to 16.
